# EUROPEAN PATENT SPECIFICATION

(11) **EP 3 946 723 B1**
(45) Date of publication and mention of the grant of the patent: **26.06.2024**
(21) Application number: 20719844.1
(22) Date of filing: 19.03.2020
(51) Int. Cl.: B01J 21/04, B01J 23/00, B01J 35/00, B01J 37/00, B01J 37/04, B01J 37/18, C07C 29/149, B01J 35/30, B01J 35/31, B01J 35/63, B01J 35/64, B01J 23/72

(54) **COPPER EXTRUDATE CATALYST AND APPLICATIONS FOR HYDROGENATION AND HYDROGENOLYSIS**
KUPFEREXTRUDATKATALYSATOR UND ANWENDUNGEN ZUR HYDRIERUNG UND HYDROGENOLYSE
CATALYSEUR D'EXTRUDAT DE CUIVRE ET APPLICATIONS D'HYDROGÉNATION ET D'HYDROGÉNOLYSE

(30) Priority: 01.04.2019 US 201962827498 P
(43) Date of publication of application: 09.02.2022
(73) Proprietor: BASF Corporation, Florham Park, NJ 07932 (US)
(72) Inventor: CHEN, Jian-Ping, Beachwood, Ohio 44122 (US); KUNDU, Arunabha, Beachwood, Ohio 44122 (US); ANGEL, Matthew, Silver Lake, Ohio 44224 (US)
(74) Representative: Altmann Stößel Dick Patentanwälte PartG mbB
(86) International application number: PCT/US2020/023643
(87) International publication number: WO 2020/205265

(56) References cited:
- JP-A- 2006 239 557
- JP-B2- 3 837 520
- JP-B2- 3 837 520
- US-A1- 2008 064 883
- US-A1- 2008 207 953
- US-A1- 2017 252 727
- US-A1- 2018 297 015

## Description

### FIELD

The present technology relates to catalysts that are useful as hydrogenolysis catalysts, and more particularly, to catalysts that are useful for hydrogenolysis of fatty acid ester compounds to form fatty alcohols. The present technology also relates to a method of preparing such catalysts and to the use of such catalysts in hydrogenolysis.

### BACKGROUND

Hydrogenolysis is a chemical reaction used for conversion of esters to alcohols, illustrated generally by the following reaction:

R-CO-OR' + 2H₂ → RCH₂-OH + R'-OH

Copper is a known catalyst for hydrogenolysis reaction. US 2017/252727 A1 relates to shaped Cu-Al-Mn catalyst bodies in extruded form, and to processes for producing them. The shaped catalyst bodies are suitable for hydrogenating organic compounds which contain a carbonyl function, more particularly for hydrogenating aldehydes, ketones, and also carboxylic acids and/or their esters.

There is a continuing need to provide catalysts that maximize desired hydrogenolysis products while eliminating by-product formation and leaching of contaminants from the catalyst into the product (*e.g*., silica). It is also desirable to provide hydrogenolysis catalysts, methods for their manufacture and methods of use, which exhibit higher catalyst activity, have longer service life, and operate at lower temperatures than existing catalysts.

### SUMMARY

In one aspect, the present invention provides a hydrogenolysis catalyst according to claim 1.

In one aspect, the present invention provides a method of making the catalyst for hydrogenolysis as defined in claim 11.

In an aspect, the present invention provides a method of conducting hydrogenolysis of fatty acid esters as defined in claim 14.

### BRIEF DESCRIPTION OF THE DRAWINGS

FIG. 1 illustrates a graph showing the %conversion to fatty alcohol of a wax ester feed using exemplary catalyst 5 and comparative reference catalyst containing silica according to the procedure in Example 2.
FIG. 2 illustrates a graph showing the cumulative intrusion ("pore volume," cm³/g) as a function of pore diameter (Å) for exemplary catalysts 1A.
FIG. 3 illustrates a graph showing the incremental pore volume as a function of pore diameter (Å) for exemplary catalyst 1A.
FIG. 4 illustrates a graph showing the X-ray powder diffraction spectra for exemplary catalyst 5.

### DETAILED DESCRIPTION

Various embodiments are described hereinafter. It should be noted that the specific embodiments are not intended as an exhaustive description or as a limitation to the broader aspects discussed herein. One aspect described in conjunction with a particular embodiment is not necessarily limited to that embodiment and may be practiced with any other embodiment(s).

As used herein, "about" will be understood by persons of ordinary skill in the art and will vary to some extent depending upon the context in which it is used. If there are uses of the term which are not clear to persons of ordinary skill in the art, given the context in which it is used, "about" will mean up to plus or minus 10% of the particular term.

The use of the terms "a" and "an" and "the" and similar referents in the context of describing the elements (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein may be performed in any suitable order unless otherwise indicated herein or otherwise clearly contradicted by context. The use of any and all examples, or exemplary language (*e.g*., "such as") provided herein, is intended merely to better illuminate the embodiments and does not pose a limitation on the scope of the claims unless otherwise stated. No language in the specification should be construed as indicating any non-claimed element as essential.

In general, "substituted" refers to an alkyl, alkenyl, cycloalkyl, or aryl group, as defined below (*e.g.,* an alkyl group) in which one or more bonds to a hydrogen atom contained therein are replaced by a bond to non-hydrogen or non-carbon atoms. Substituted groups also include groups in which one or more bonds to a carbon(s) or hydrogen(s) atom are replaced by one or more bonds, including double or triple bonds, to a heteroatom. Thus, a substituted group will be substituted with one or more substituents, unless otherwise specified. In some embodiments, a substituted group is substituted with 1, 2, 3, 4, 5, or 6 substituents. Examples of substituent groups include: halogens (*i.e.*, F, Cl, Br, and I); hydroxyls; alkoxy, alkenoxy, alkynoxy, aryloxy, aralkyloxy, heterocyclyloxy, and heterocyclylalkoxy groups; carbonyls (oxo); carboxyls; esters; urethanes; oximes; hydroxylamines; alkoxyamines; aralkoxyamines; thiols; sulfides; sulfoxides; sulfones; sulfonyls; sulfonamides; amines; N-oxides; hydrazines; hydrazides; hydrazones; azides; amides; ureas; amidines; guanidines; enamines; imides; isocyanates; isothiocyanates; cyanates; thiocyanates; imines; nitro groups; nitriles (*i.e.,* CN); and the like.

As used herein, "alkyl" groups include straight chain and branched alkyl groups having from 1 to about 20 carbon atoms, and typically from 1 to 12 carbons or, in some embodiments, from 1 to 8 carbon atoms. As employed herein, "alkyl groups" include cycloalkyl groups as defined below. Alkyl groups may be substituted or unsubstituted. Examples of straight chain alkyl groups include methyl, ethyl, n-propyl, n-butyl, n-pentyl, n-hexyl, n-heptyl, and n-octyl groups. Examples of branched alkyl groups include, but are not limited to, isopropyl, sec-butyl, t-butyl, neopentyl, and isopentyl groups. Representative substituted alkyl groups may be substituted one or more times with, for example, amino, thio, hydroxy, cyano, alkoxy, and/or halo groups such as F, Cl, Br, and I groups. As used herein the term haloalkyl is an alkyl group having one or more halo groups. In some embodiments, haloalkyl refers to a per-haloalkyl group.

Alkenyl groups are straight chain, branched or cyclic alkyl groups having 2 to about 20 carbon atoms, and further including at least one double bond. In some embodiments alkenyl groups have from 2 to 12 carbons, or, typically, from 2 to 8 carbon atoms. Alkenyl groups may be substituted or unsubstituted. Alkenyl groups include, for instance, vinyl, propenyl, 2-butenyl, 3-butenyl, isobutenyl, cyclohexenyl, cyclopentenyl, cyclohexadienyl, butadienyl, pentadienyl, and hexadienyl groups among others. Alkenyl groups may be substituted similarly to alkyl groups. Divalent alkenyl groups, *i.e*., alkenyl groups with two points of attachment, include, but are not limited to, CH-CH=CH₂, C=CH₂, or C=CHCH₃.

Reference to an "alkali metal component" means a material used to deliver an alkali metal, for example metal oxides, hydroxides, or carbonates, which may be in powder form or in an aqueous solution.

Reference to "binder" or "binder system" means a material suitable for binding components together to form a catalyst in a shape. Generally, the binder component is extrudable and used to form extruded catalysts and/or the binder component is able to form tableted catalysts. Thus, the binder or binder system, may include zirconium oxide, alumina, and mixtures thereof.

All references to pore diameters and pore volumes in the specification and claims of this application are based upon measurements utilizing mercury porosimetry. A typical method is described by R. Anderson, Experimental Methods in Catalytic Research, Academic Press, New York, 1968. The pore volumes are determined utilizing the catalysts in their oxide forms. That is, the pore diameters and pore volumes reported herein are obtained for the catalyst after calcination, but prior to any reduction of the oxide. Those skilled in the art often refer to the catalyst containing the metal oxides as the "oxide" or "oxide precursor" form of the catalyst.

The catalysts of the present technology are based upon a calcined coprecipitated mixture of copper oxide-alumina oxide and manganese oxide and zirconium acetate solution. Formation of the catalysts includes mixing and extruding of the precursor materials, followed by drying and calcination. Other additives may be included in the mixtures to form the catalysts.

The copper extrudate catalysts described herein exhibit improved catalytic hydrogenolysis activity over and equivalent selectivity for fatty alcohol processes than current commercially available catalysts that include commonly used binders (*e.g*., calcium silicate, sodium silicate, silica sol, clay, etc.). These commonly used binders contain silica that may be leached out under reaction conditions, especially for fatty alcohol that impact product quality by the silica purity. The catalysts of the present technology as described herein, include zirconium based binder system (*e.g.*, ZrO₂ or ZrO₂-Al₂O₃) and do not contain silica that could be leached to alcohol product in fatty alcohol production processes.

Surprisingly, the inventors discovered the hydrogenolysis catalysts of the present technology exhibit strong mechanical strength, high activity for hydrogenation/hydrogenolysis reactions for fatty alcohol production, and improved selectivity across a broader operation temperature range compared to current commercial catalysts.

Provided are hydrogenolysis catalysts that are useful for the production of fatty alcohols from fatty acid esters. Methods of making and using the same are also provided. These catalysts are formed from a catalytic component and a binder, which are processed together, for example, by extrusion or by tableting, to form the catalyst. The catalytic component includes a copper oxide, manganese oxide, and aluminum oxide. The binder includes a zirconium component (*e.g*., ZrO₂ or ZrO₂-Al₂O₃).

In the present description:
1 psi = 6895 Pa
1 inch = 2.54 cm
1 lb = 0.454 kg

### Catalyst Composition

In one aspect, a hydrogenolysis catalyst of the invention is as defined in claim 1.

In any embodiment herein, the hydrogenolysis catalyst may be substantially free of silicon or an oxide thereof. In addition, the hydrogenolysis catalysts may be free of silicon or an oxide thereof in order to reduce exposure to such material. As used herein, the hydrogenolysis catalyst may be free of such materials if their presence is in an amount that does not materially affect the physical, chemical and catalytic characteristics of the catalysts when compared to those which are completely free of such materials. In the catalyst of the invention, the amount of such materials is not greater than about 1.5% by weight, more preferably not greater than 0.5% weight. The phrase "substantially free of silicon or oxides thereof' refers to less than about 1.5 wt%, less than about 1 wt%, less than about 0.5 wt%, less than about 0.1 wt%, less than about 0.01 wt%, or 0 wt% based on the total weight of the hydrogenolysis catalyst. In any embodiment herein, the hydrogenolysis catalyst may be free of silicon or an oxide thereof.

The catalyst comprises copper oxide in an amount from about 45 wt% to about 55 wt%. For example, the amount of the copper oxide may include, but is not limited to, about 45 wt%, about 50 wt%, about 55 wt%, or any range including and/or in between any two of the preceding values.

In any embodiment herein, the hydrogenolysis catalyst includes manganese oxide in an amount from about 0 wt% to about 10 wt% by weight of the hydrogenolysis catalyst. Suitable amounts of the manganese oxide include, but are not limited to, from about 0 wt% to about 10 wt%, about 3 wt% to about 10 wt%, about 5 wt% to about 10 wt%, about 6 wt% to about 9 wt%, or any range including and/or in between any two of the preceding values. For example, the manganese oxide may be present in an amount of about 1 wt%, about 2 wt%, about 3 wt%, about 4 wt%, about 5 wt%, about 6 wt%, about 7 wt%, about 8 wt%, about 9 wt%, about 10 wt%, or any range including and/or in between any two of the preceding values.

In any embodiment herein, the hydrogenolysis catalyst includes aluminum oxide in an amount from about 15 wt% to about 40 wt% by weight of the hydrogenolysis catalyst. Suitable amounts of the aluminum oxide may include, but are not limited to, from about 15 wt% to about 40 wt%, about 20 wt% to about 35 wt%, about 25 wt% to about 35 wt%, or any range including and/or in between any two of the preceding values. For example, the aluminum oxide may be present in an amount of about 15 wt%, about 20 wt%, about 25 wt%, about 30 wt%, about 35 wt%, about 40 wt%, or any range including and/or in between any two of the preceding values.

The hydrogenolysis catalyst includes a binder, where the binder includes a zirconium component. In any embodiment herein, the zirconium component may be present in the reduced metal or oxide forms or as a precursor to such forms and in one or more oxidation states as discussed above. For example, the zirconium component is present in the form of zirconium oxide (*e.g.*, ZrO₂). The zirconium component is present in an amount from about 4 wt% to about 15 wt% by weight of the hydrogenolysis catalyst The zirconium component is present in an amount from about 4 wt% to about 15 wt%, about 5 wt% to about 12 wt%, about 5 wt% to about 8 wt%, or any range including and/or in between any two of the preceding values. For example, the zirconium component may be present in an amount of about 4 wt%, about 5 wt%, about 6 wt%, about 7 wt%, about 8 wt%, about 9 wt%, about 10 wt%, about 11 wt%, about 12 wt%, about 13 wt%, about 14 wt%, about 15 wt%, or any range including and/or in between any two of the preceding values.

In any embodiment herein, the binder may further include alumina. For example, the binder may be present as a zirconium oxide - aluminum oxide (ZrO₂-Al₂O₃) binder system.

In any embodiment herein, the hydrogenolysis catalyst may include from about 45% to about 55% of copper oxide, about 5 wt% to about 12 wt% manganese oxide, about 20 wt% to about 35 wt% aluminum oxide, and about 5 wt% to about 12 wt% zirconium oxide.

The hydrogenolysis catalyst as described herein in any embodiment may further include an alkali metal component. In any embodiment herein, the alkali metal is selected from the group consisting of sodium (Na), potassium (K), rubidium (Rb), cesium (Cs), and combinations thereof. These metals may be present in the reduced metal or oxide forms or as precursors to such forms and in one or more oxidation states as discussed above. For example, the alkali metal component may include sodium in the form of disodium oxide. In any embodiment herein, the alkali metal may be present in an amount from about 0 wt% to about 1 wt% by weight of the hydrogenolysis catalyst. For example, the alkali metal component may be present in an amount of about 0.01 wt%, 0.05 wt%, about 0.1 wt%, about 0.2 wt%, about 0.3 wt%, about 0.4 wt%, about 0.5 wt%, about 0.6 wt%, about 0.7 wt%, about 0.8 wt%, about 0.9 wt%, about 1 wt%, or any range including and/or in between any two of the preceding values.

The hydrogenolysis catalysts may be provided as tablets or extrudates. One way to process the blend of all of the ingredients is to extrude it through a shaping orifice to form an extruded catalyst body, or extrudate. Other catalyst bodies may be shaped into spheres or any other convenient formation. Another way is to tablet the catalysts. For example, the hydrogenolysis catalyst may be extruded or tableted in sizes including, but not limited to, 1/8" by 1/8", 3/16" by 3/16", 1/4" by 1/4", 3/16" by 1/4", 1/4" by 1/16", or 1/8" by 1/16".

In any embodiment herein, the hydrogenolysis catalyst may be provided in tableted or extruded form, and may exhibit a crush strength in an amount from about 1.0 lbs/mm to about 6.0 lbs/mm. For example, the hydrogenolysis catalyst may exhibit a side crush strength of about 1.0 lbs/mm, about 1.5 lbs/mm, about 2.0 lbs/mm, about 2.5 lbs/mm, about 3.0 lbs/mm, about 3.5 lbs/mm, about 4.0 lbs/mm, about 4.5 lbs/mm, about 5.0 lbs/mm, about 5.5 lbs/mm, about 6.0 lbs/mm, or any range including and/or in between any two of the preceding values. In some embodiments, the catalyst may exhibit a side crush strength from about 3.5 lbs/mm to about 5.0 lbs/mm. In any embodiment, the hydrogenolysis catalyst after use in one or more catalytic process (*i.e*., spent catalyst) may exhibit a crush strength as described herein.

In any embodiment herein, the catalyst is a calcined and extruded catalyst.

Specifically, these hydrogenolysis catalysts contain a significant amount of mesoporosity. Reference to "mesoporosity" or "mesopore" means those pores having a pore diameter in the range of about 50 to about 1000 Angstroms (Å). For example, the hydrogenolysis catalysts may have a pore diameter from about 50 Å to about 1000 Å, about 75 Å to about 1000 Å, about 100 Å to about 900 Å, about 150 Å to about 850 Å, about 300 Å to about 800 Å, or any range including and/or in between any two of the preceding values.

In any embodiment herein, the hydrogenolysis catalyst may have a pore volume that is greater than or equal to 0.2 cm³/g. For example, the hydrogenolysis catalyst may have a pore volume of about 0.2 cm³/g, about 0.25 cm³/g, about 0.3 cm³/g, about 0.35 cm³/g, about 0.4 cm³/g, about 0.45 cm³/g, about 0.5 cm³/g, about 0.55 cm³/g, about 0.6 cm³/g, about 0.65 cm³/g, about 0.7 cm³/g, about 0.75 cm³/g, about 0.8 cm³/g, or any range including and/or in between any two of the preceding values.

In any embodiment herein, the hydrogenolysis catalyst may have a packed bulk density of about 0.8 g/cm³ to about 1.5 g/cm³. For example, the hydrogenolysis catalyst may have a packed bulk density of about 0.8 g/cm³, about 0.9 g/cm³, about 1.0 g/cm³, about 1.2 g/cm³, about 1.3 g/cm³, about 1.4 g/cm³, about 1.5 g/cm³, or any range including and/or in between any two of the preceding values. In any embodiment herein, the hydrogenolysis catalyst may have a packed bulk density of about 0.8 g/cm³ to about 1.5 g/cm³, about 0.8 g/cm to about 1 g/cm³, about 0.8 g/cm³ to about 0.95 g/cm³, or any range including and/or in between any two of the preceding values. In any embodiment herein, the hydrogenolysis catalyst may be in tablet form and have a packed bulk density of about 1.0 g/cm³, about 1.1 g/cm³, about 1.2 g/cm³, about 1.3 g/cm³, about 1.4 g/cm³, about 1.5 g/cm³, or any range including and/or in between any two of the preceding values.

In any embodiment herein, the hydrogenolysis catalyst may have a Brunauer-Emmett-Teller ("BET") surface area of about 15 m²/g to about 70 m²/g. For example, the hydrogenation catalyst has a BET surface area of about 15 m²/g, about 20 m²/g, about 25 m²/g, about 30 m²/g, about 35 m²/g, about 40 m²/g, about 45 m²/g, about 50 m²/g, about 55 m²/g, about 60 m²/g, about 65 m²/g, about 70 m²/g, or any range including and/or in between any two of the preceding values. In any embodiment herein, the hydrogenation catalyst has a BET surface area of about 15 m²/g to about 70 m²/g, about 25 m²/g to about 65 m²/g, about 45 m²/g to about 60 m²/g, about 50 m²/g to about 60 m²/g, or any range including and/or in between any two of the preceding values.

In any embodiment herein, the hydrogenolysis catalyst is in an unreduced form and exhibits an X-ray powder diffraction profile with 2θ peaks at 31.1°, 36.0°, 36.8°, 38.8°, 44.6°, 48.8°, 61.7°, 64.9°, 68.1°. In any embodiment herein, the hydrogenolysis catalyst is in an unreduced form and exhibits an X-ray powder diffraction profile with 2θ peaks at 18.9°, 31.1°, 32.5°, 36.0°, 36.8°, 38.8°, 44.6°, 48.8°, 53.6°, 58.3°, 59.1°, 61.7°, 64.9°, 66.8°, 68.1°, 72.2°, 75.1°, and 77.0°.

In any embodiment herein, the hydrogenolysis catalyst has a copper oxide crystallite size of about 60 Å to about 250 Å. For example, the calcined hydrogenolysis catalyst may have a copper oxide crystallite size of about 60 Å, about 65 Å, about 70 Å, about 75 Å, about 80 Å, about 85 Å, about 90 Å, about 95 Å, about 100 Å, about 105 Å, about 110 Å, about 120 Å, about 125 Å, about 130 Å, about 135 Å, about 140 Å, about 145 Å, about 150 Å, about 155 Å, about 160 Å, about 165 Å, about 170 Å, about 175 Å, about 180 Å, about 185 Å, about 190 Å, about 195 Å, about 200 Å, about 205 Å, about 210 Å, about 215 Å, about 220 Å, about 225 Å, about 230 Å, about 235 Å, about 240 Å, about 245 Å, about 250 Å, or any range including and/or in between any two of the preceding values. In any embodiment, the calcined hydrogenation catalyst may have a copper oxide crystallite size of about 200 Å to about 220 Å.

### Method of Making

In one aspect, methods of making the catalyst for hydrogenolysis described herein is provided. The method of the invention is as defined in claim 11.

In any embodiment herein, the catalytic component includes a mixture of copper oxide, manganese oxide, and aluminum oxide provided in a the form of a pre-calcined powder. The pre-calcined powder may be calcined at a temperature from about 300°C to about 750°C. Suitable calcination temperatures may include, but are not limited to, about 300°C, about 350°C, about 400°C, about 450°C, about 500°C, about 550°C, about 600°C, about 650°C, about 700°C, about 750°C, or any range including and/or in between any two of the preceding values.

In any embodiment herein, the zirconium component may include zirconium acetate. In any embodiment herein, the binder may further include alumina.

In any embodiment herein, the method may further including mixing one or more extrusion aids in to the material mixture. Suitable extrusion aids may include, but are not limited to, a polymeric polysaccharide, hydroxypropyl methyl cellulose, hydroxylethyl methyl cellulose, or a mixture of two or more thereof. For example, the extrusion aid may be selected from a commercially available extrusion aid, including but not limited to, Zusoplast, Methocel, Walocel, or a mixture thereof.

The method includes calcining the material mixture at a temperature, and for a time, sufficient to cure form a calcined hydrogenolysis catalyst. In any embodiment herein, the calcining may occur at a temperature from about 200°C to about 1000°C. For example, the calcining may occur at a temperature of about 200°C, about 250°C, about 300°C, about 350°C, about 400°C, about 450°C, about 500°C, about 550°C, about 600°C, about 650°C, about 700°C, about 750°C, about 800°C, about 850°C, about 900°C, about 950°C, about 1000°C, or any range including and/or in between any two of the preceding values. In any embodiment herein, the calcining temperature may be from about 400°C to about 650°C, from about 400°C to about 550°C, or from about 450°C to about 500°C. In any embodiment herein, the calcination may occur over a period from about 0.5 h to about 4 h. In any embodiment, the calcination may occur over a period of about 0.5 h, about 1 h, about 1.5 h, about 2 h, about 2.5 h, about 3 h, about 3.5 h, about 4 h, or any range including and/or in between any two of the preceding values.

In any embodiment herein, forming the material mixture may be by extrusion, tableting, or spherization. In any embodiment herein, the forming may be extruding or tableting the material mixture to obtain the formed material mixture. For example, the formed material mixture may be extruded or tableted in sizes including, but not limited to, 1/8" by 1/8", 3/16" by 3/16", 1/4" by 1/4", 3/16" by 1/4", 1/4" by 1/16", or 1/8" by 1/16". In any embodiment herein, the forming may include spherization of the material mixture to obtain the formed material mixture.

In any embodiment herein, the hydrogenolysis catalyst provided in tableted or extruded form, may exhibit a crush strength in an amount from about 1.0 lbs/mm to about 6.0 lbs/mm. For example, the catalyst may exhibit a side crush strength of about 1.0 lbs/mm, about 1.5 lbs/mm, about 2.0 lbs/mm, about 2.5 lbs/mm, about 3.0 lbs/mm, about 3.5 lbs/mm, about 4.0 lbs/mm, about 4.5 lbs/mm, about 5.0 lbs/mm, about 5.5 lbs/mm, about 6.0 lbs/mm, or any range including and/or in between any two of the preceding values. In some embodiments, the catalyst may exhibit a side crush strength from about 3.5 lbs/mm to about 5.0 lbs/mm.

The method may further include removing at least some of the water from the wet material mixture prior to calcining. For example, the removing may include drying the wet material mixture. In any embodiment herein, the removing may be conducted at a temperature including, but not limited to, from about 40°C to about 150°C. For example, the removing may occur at a temperature of about 40°C, about 50°C, about 60 °C, about 70°C, about 80°C, about 90°C, about 100°C, about 110°C, about 120°C, about 130°C, about 140°C, about 150°C, or any range including and/or in between any two of the preceding values.

The calcined hydrogenolysis catalyst includes copper oxide in an amount of about 45 wt% to about 55 wt%. For example, the amount of the copper oxide may include, but is not limited to, about 45 wt%, about 50 wt%, about 55 wt%, or any range including and/or in between any two of the preceding values.

In any embodiment herein, the calcined hydrogenolysis catalyst includes manganese oxide in an amount from about 0 wt% to about 10 wt% by weight of the calcined hydrogenolysis catalyst. Suitable amounts of the manganese oxide include, but are not limited to, from about 0 wt% to about 10 wt%, about 3 wt% to about 10 wt%, about 5 wt% to about 10 wt%, about 6 wt% to about 9 wt%, or any range including and/or in between any two of the preceding values. For example, the manganese oxide may be present in an amount of about 1 wt%, about 2 wt%, about 3 wt%, about 4 wt%, about 5 wt%, about 6 wt%, about 7 wt%, about 8 wt%, about 9 wt%, about 10 wt%, or any range including and/or in between any two of the preceding values.

In any embodiment herein, the calcined hydrogenolysis catalyst includes aluminum oxide in an amount from about 15 wt% to about 40 wt% by weight of the calcined hydrogenolysis catalyst. Suitable amounts of the aluminum oxide may include, but are not limited to, from about 15 wt% to about 40 wt%, about 20 wt% to about 35 wt%, about 25 wt% to about 35 wt%, or any range including and/or in between any two of the preceding values. For example, the aluminum oxide may be present in an amount of about 15 wt%, about 20 wt%, about 25 wt%, about 30 wt%, about 35 wt%, about 40 wt%, or any range including and/or in between any two of the preceding values.

The zirconium component is present in the calcined hydrogenolysis catalyst in an amount from about 4 wt% to about 15 wt% by weight of the calcined hydrogenolysis catalyst. The zirconium component is present in an amount from about 4 wt% to about 15 wt%, about 5 wt% to about 12 wt%, about 5 wt% to about 8 wt%, or any range including and/or in between any two of the preceding values. For example, the zirconium component may be present in an amount of about 4 wt%, about 5 wt%, about 6 wt%, about 7 wt%, about 8 wt%, about 9 wt%, about 10 wt%, about 11 wt%, about 12 wt%, about 13 wt%, about 14 wt%, about 15 wt%, or any range including and/or in between any two of the preceding values.

In any embodiment herein, the binder may further include alumina. For example, the binder may be present as a zirconium oxide - aluminum oxide (ZrO₂-Al₂O₃) binder system.

In any embodiment herein, the calcined hydrogenolysis catalyst may include from about 45% to about 55% of copper oxide, about 5 wt% to about 12 wt% manganese oxide, about 20 wt% to about 35 wt% aluminum oxide, and about 5 wt% to about 12 wt% zirconium oxide.

The calcined hydrogenolysis catalysts may have a pore diameter in the range of about 150 to about 1000 Angstroms (Å). For example, the calcined hydrogenolysis catalysts may have a pore diameter from about 150 Å to about 1500 Å, about 200 Å to about 1000 Å, about 225 Å to about 800 Å, about 250 Å to about 700 Å, about 300 Å to about 600 Å, or any range including and/or in between any two of the preceding values.

In any embodiment herein, the calcined hydrogenolysis catalyst may have a pore volume that is greater than or equal to 0.2 cm³/g. For example, the calcined hydrogenolysis catalyst may have a pore volume of about 0.2 cm³/g, about 0.25 cm³/g, about 0.3 cm³/g, about 0.35 cm³/g, about 0.4 cm³/g, about 0.45 cm³/g, about 0.5 cm³/g, about 0.55 cm³/g, about 0.6 cm³/g, about 0.65 cm³/g, about 0.7 cm³/g, about 0.75 cm³/g, about 0.8 cm³/g, or any range including and/or in between any two of the preceding values.

In any embodiment herein, the calcined hydrogenolysis catalyst may have a packed bulk density of about 0.8 g/cm³ to about 1.5 g/cm³. For example, the calcined hydrogenolysis catalyst may have a packed bulk density of about 0.8 g/cm³, about 0.9 g/cm³, about 1.0 g/cm³, about 1.1 g/cm³, about 1.2 g/cm³, about 1.3 g/cm³, about 1.4 g/cm³, about 1.5 g/cm³, or any range including and/or in between any two of the preceding values. In any embodiment herein, the calcined hydrogenolysis catalyst may have a packed bulk density of about 0.8 g/cm³ to about 1.5 g/cm³, about 0.8 g/cm to about 1 g/cm³, about 0.8 g/cm³ to about 0.95 g/cm³, or any range including and/or in between any two of the preceding values. In any embodiment herein, the calcined hydrogenolysis catalyst may be in tablet form and have a packed bulk density of about 1.0 g/cm³, about 1.1 g/cm³, about 1.2 g/cm³, about 1.3 g/cm³, about 1.4 g/cm³, about 1.5 g/cm³, or any range including and/or in between any two of the preceding values.

In any embodiment herein, the calcined hydrogenolysis catalyst may have a BET surface area of about 15 m²/g to about 70 m²/g. For example, the calcined hydrogenation catalyst has a BET surface area of about 15 m²/g, about 20 m²/g, about 25 m²/g, about 30 m²/g, about 35 m²/g, about 40 m²/g, about 45 m²/g, about 50 m²/g, about 55 m²/g, about 60 m²/g, about 65 m²/g, about 70 m²/g, or any range including and/or in between any two of the preceding values. In any embodiment herein, the calcined hydrogenation catalyst has a BET surface area of about 15 m²/g to about 70 m²/g, about 25 m²/g to about 65 m²/g, about 45 m²/g to about 60 m²/g, about 50 m²/g to about 60 m²/g, or any range including and/or in between any two of the preceding values.

In any embodiment herein, the hydrogenolysis catalyst is in an unreduced form and exhibits an X-ray powder diffraction profile with 2θ peaks at 31.1°, 36.0°, 36.8°, 38.8°, 44.6°, 48.8°, 61.7°, 64.9°, 68.1°. In any embodiment herein, the hydrogenolysis catalyst is in an unreduced form and exhibits an X-ray powder diffraction profile with 2θ peaks at 18.9°, 31.1°, 32.5°, 36.0°, 36.8°, 38.8°, 44.6°, 48.8°, 53.6°, 58.3°, 59.1°, 61.7°, 64.9°, 66.8°, 68.1°, 72.2°, 75.1°, and 77.0°.

In any embodiment herein, the calcined hydrogenolysis catalyst has a copper oxide crystallite size of about 60 Å to about 250 Å. For example, the calcined hydrogenolysis catalyst may have a copper oxide crystallite size of about 60 Å, about 65 Å, about 70 Å, about 75 Å, about 80 Å, about 85 Å, about 90 Å, about 95 Å, about 100 Å, about 105 Å, about 110 Å, about 120 Å, about 125 Å, about 130 Å, about 135 Å, about 140 Å, about 145 Å, about 150 Å, about 155 Å, about 160 Å, about 165 Å, about 170 Å, about 175 Å, about 180 Å, about 185 Å, about 190 Å, about 195 Å, about 200 Å, about 205 Å, about 210 Å, about 215 Å, about 220 Å, about 225 Å, about 230 Å, about 235 Å, about 240 Å, about 245 Å, about 250 Å, or any range including and/or in between any two of the preceding values. In any embodiment, the calcined hydrogenolysis catalyst may have a copper oxide crystallite size of about 200 Å to about 220 Å.

In another aspect, the present technology provides a catalyst for hydrogenolysis as prepared by the method described herein in any embodiment.

### Methods of Use

In an aspect of the invention, a method of conducting hydrogenolysis of fatty acid esters is provided, where the method is as defined in claim 14.

In any embodiment herein, the fatty acid ester may be a compound of Formula I:

R¹-CO-OR² (I)

wherein R¹ and R² are each independently a branched or unbranched C₁ to C₂₄ alkyl or a branched or unbranched C₂ to C₂₄ alkenyl.

In any embodiment herein, R¹ and R² may each independently be a C₁₀ to C₁₄ branched or unbranched alkyl or a C₁₀ to C₁₄ branched or unbranched alkenyl. In any embodiment herein, R¹ and R² may each independently be a C₁₂ to C₁₄ branched or unbranched alkyl. In some embodiments herein, when R¹ is a C₁₀ to Cis branched or unbranched alkyl or a C₁₀ to Cis branched or unbranched alkenyl, R² may be a C₁ to C₆ alkyl. For example, R² may be a C₁ to C₄ alkyl. In any embodiment herein, R² may be a methyl.

In any embodiment herein, R¹ and R² together may be represented by a C₁ to C₂₄ alkyl or C₁ to C₂₄ alkenyl such that the compound represented by Formula (I) forms a wax ester having a total of 12 to 36 carbon atoms.

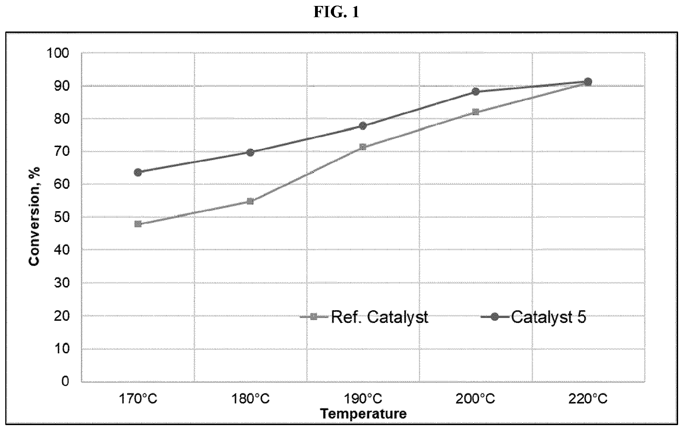

The hydrogenolysis catalysts of the present technology exhibits improved activity and selectivity for fatty alcohol formation at lower temperatures compared to current commercially available catalyst, such as catalyst containing silicon or oxides thereof (FIG. 1). In any embodiment herein, the method may be conducted at a temperature of at least about 170°C. For example, the temperature may be from about 170°C to about 250°C, about 170°C to about 220°C, or about 170°C to about 180°C. Suitable temperatures include, but are not limited to, about 170°C, about 175°C, about 180°C, about 185°C, about 190°C, about 195°C, about 200°C, about 205°C, about 210°C, about 215°C, about 220°C, about 225°C, about 230°C, about 235°C, about 240°C, about 245°C, about 250°C, or any range including and/or in between any two of the preceding values.

The present invention, thus generally described, will be understood more readily by reference to the following examples, which are provided by way of illustration and are not intended to be limiting of the present invention.

### EXAMPLES

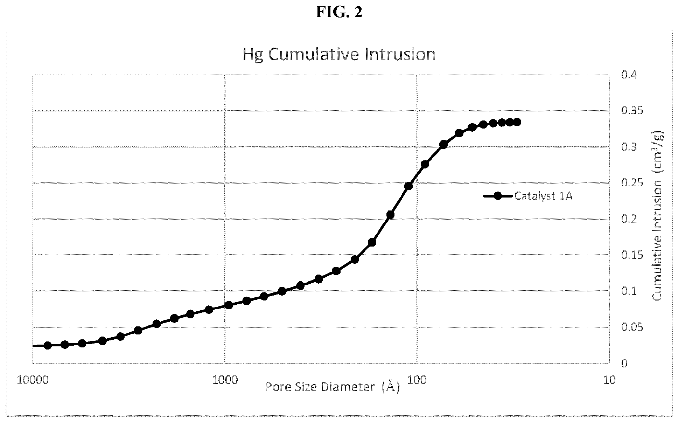

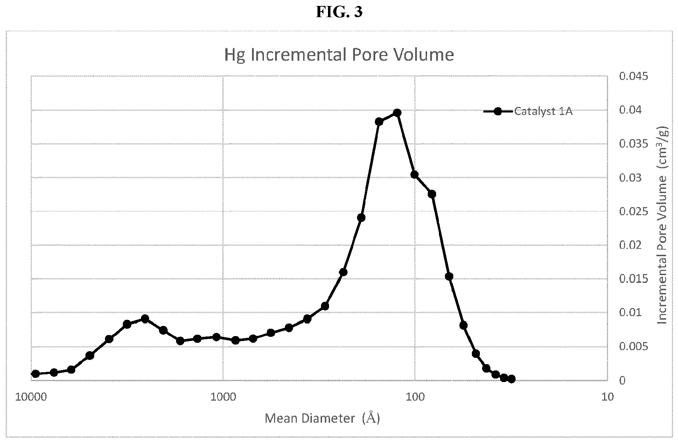

**Example 1-5:** A series of copper catalysts having varying levels of copper oxide, manganese oxide, aluminum oxide, and zirconium oxide were prepared as follows. Calcined copper manganese aluminum powder, zirconium acetate, alumina, organic binder (i.e., Zusoplast), and water were mixed and kneaded. The mixture was then extruded with an extruder and dried. The extrudates were then calcined at 250° to 1000°C. As shown in FIG. 2, the exemplary catalyst 1A exhibited pore volumes of about 0.1-0.35 cm³/g at pore diameters ranging from 100-1000 Å. FIG. 3 shows exemplary catalyst 1A exhibited a mesoporosity between 50 to 1000 Å. The catalysts had the properties outlined in Table 1, where "3F" means 3-fluted or tri-lobe.

Catalysts 1E, 3A, 4A, 5, and a reference catalyst containing copper on silica were characterized by temperature-programmed-reduction and Cu metal dispersion. The results are summarized in the table below.

| **Catalyst** | **Temperature-Programed Reduction (TPR) Peak Temperature, °C** | **Dispersion, %** |
|---|---|---|
| 1E | 140 | 2.4 |
| 3A | 132 | 2.4 |
| 4A | 132 | 2.3 |
| 5 | 136 | 2.6 |
| Ref. Catalyst | 155 | 1.8 |

Temperature-programmed-reduction peak temperatures is an indication of easiness of catalyst reduction by hydrogen. Lower peak temperature catalysts will be reduced easier under the same reduction conditions. As shown in the table above, Catalysts 1E, 3A, 4A, and 5 exhibited lower peak temperatures than the reference catalyst. Also, Catalysts 1E, 3A, 4A, and 5 exhibited higher copper metal dispersion than the reference catalyst. Copper metal dispersion is the ratio of surface copper metal to the total copper metal. Higher copper dispersion means smaller copper metal crystallite size that generally will have higher catalytic activity. These will be further demonstrated in Examples 6 and 7 for the hydrogenolysis activities of esters.

***XRD Analysis:*** A PANalytical MPD X'Pert Pro diffraction system was used to collect data for exemplary catalysts 5. Cu K_{α} radiation was used in the analysis with generator settings of 45kV and 40mA. The optical path consisted of a 1° divergence slit, 2° anti-scatter slit, the sample, and an X'Celerator position sensitive detector. Each catalyst sample was first prepared by backpacking the sample into round mount. The data collection from the round mount covered a range from 10° to 90° 2θ using a step scan with a step size of 0.017° 2θ and a scan speed of 0.036° 2θ per second. The X'Pert Pro HighScore program was used for phase identification analysis.

As shown below, monoclinic and/or cubic copper oxide (CuO/Cu₂O) were the predominant phases for exemplary catalyst 5.

### Phase Determination

| **Ex.** | **Phase Match Candidate** | **Semi-quantitative (wt%)** | **^{†}ICDD Ref.** |
|---|---|---|---|
| 5 | monoclinic copper oxide (CuO) | 47 | 4-008-2756 |
| | cubic copper aluminum oxide (CuAl₂O₄) | 40 | 4-011-8980 |
| | cubic copper manganese oxide (Cu₃Mn₃O₈) | 7 | 4-007-6753 |
| | cubic zirconium oxide (ZrO₂) | 6 | 4-013-3441 |

| | | | |
|---|---|---|---|
| *Reduced catalyst. ^{†}International Centre for Diffraction Data (ICDD) | | | |

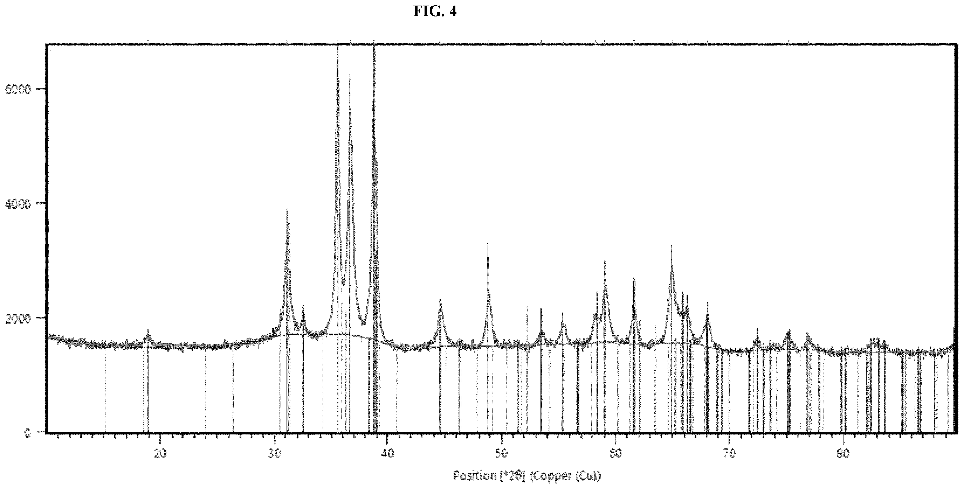

The full listing of 2θ peaks in FIG. 4 is 18.9°, 31.1°, 32.5°, 36.0°, 36.8°, 38.8°, 44.6°, 48.8°, 53.6°, 58.3°, 59.1°, 61.7°, 64.9°, 66.8°, 68.1°, 72.2°, 75.1°, and 77.0°.

**Table 1. Catalysts 1-5**

| Example | %CuO | % Al₂O₃ | % MnO | % ZrO₂ | % Na₂O | Size (3F) | Packed bulk density (g/cc) | Crush strength, lbs/mm | Calcination Temp. (°C) |
|---|---|---|---|---|---|---|---|---|---|
| 1A | 51.7 | 29.5 | 8.8 | 7.3 | 0.4 | 1/16" | 1.0 | -- | 500 |
| 1B | 51.7 | 29.5 | 8.8 | 7.3 | 0.4 | 1/16" | -- | -- | 400 |
| 1C | 51.7 | 29.5 | 8.8 | 7.3 | 0.4 | 1/16" | 1.0 | -- | 500 |
| *1D | 51.7 | 29.5 | 8.8 | 7.3 | 0.4 | 1/16" | 1.0 | - | 500 |
| 1E | 51.7 | 29.5 | 8.8 | 7.3 | 0.4 | 1/16" | 1.0 | -- | 500 |
| *1F | 51.7 | 29.5 | 8.8 | 7.3 | 0.4 | 1/16" | 1.0 | -- | 500 |
| 3A | 52.2 | 29.9 | 8.8 | 8.3 | 0.4 | 1/16" | 1.0 | 3.5 | 500 |
| *3B | 52.2 | 29.9 | 8.8 | 8.3 | 0.4 | 1/16" | 1.0 | 3.5 | 500 |
| 3C | 52.2 | 29.9 | 8.8 | 8.3 | 0.4 | 1/16" | 1.0 | 3.5 | 500 |
| *3D | 52.2 | 29.9 | 8.8 | 8.3 | 0.4 | 1/16" | 1.0 | 3.5 | 500 |
| 4A | 51.2 | 29.7 | 8.6 | 8.1 | 0.4 | 1/16" | -- | 4.0 | 500 |
| 4B | 51.2 | 29.7 | 8.6 | 8.1 | 0.4 | 1/16" | 1.1 | 4.0 | 500 |
| *4C | 51.2 | 29.7 | 8.6 | 8.1 | 0.4 | 1/16" | 1.1 | 4.0 | 500 |
| 4D | 51.2 | 29.7 | 8.6 | 8.1 | 0.4 | 1/16" | 1.1 | 4.0 | 500 |
| *4E | 51.2 | 29.7 | 8.6 | 8.1 | 0.4 | 1/16" | 1.1 | 4.0 | 500 |
| 4F | 51.2 | 29.7 | 8.6 | 8.1 | 0.4 | 1/16" | 1.1 | 4.0 | 500 |
| *4G | 51.2 | 29.7 | 8.6 | 8.1 | 0.4 | 1/16" | 1.1 | 4.0 | 500 |
| 4H | 51.2 | 29.7 | 8.6 | 8.1 | 0.4 | 1/16" | 1.1 | 4.0 | 500 |
| *4I | 51.2 | 29.7 | 8.6 | 8.1 | 0.4 | 1/16" | 1.1 | 4.0 | 500 |
| 5 | 52.0 | 30.4 | 8.9 | 7.2 | 0.4 | 1/16" | 1.0 | -- | 500 |

| | | | | | | | | | |
|---|---|---|---|---|---|---|---|---|---|
| *Reduced catalyst | | | | | | | | | |

**EXAMPLE 6:** Fatty Acid Wax Ester Hydrogenolysis Testing in a liquid phase process. For liquid phase wax ester hydrogenolysis catalyst testing, 100 cc of catalyst extrudates as described in Example 1 was loaded into a 1.25" ID stainless steel fixed-bed down flow reactor. An equal volume of inert 14 X 28 mesh α-alumina granules was loaded with the catalyst and served as interstitial packing. The reactor was also equipped with a 3/16" thermowell that housed six thermocouples, one at the inlet and five spaced equally throughout the catalyst bed which was approximately 8" in length. The reactor and heated via furnace jacketed around the reactor tube.

Once loaded, the reactor was purged with 2 standard liter per minute (slpm) N₂ for approximately 1 hour to remove air and subsequently heated to 190 °C under flowing N₂. The catalyst was activated by introducing H₂ in a stepwise fashion at atmospheric pressure. Once the reduction was complete, the temperature was ramped down to 170°C in 100 standard liter per hour (slph) hydrogen. The temperature was stabilized at 170°C, and the reactor was pressurized from atmospheric pressure to 1015 psig.

The commercial wax ester feed (C₁₂-C₁₄ wax ester) was then pumped into the reactor at a rate of 63 g/hr to give an overall LHSV of about 0.73 hr⁻¹. The hydrogen:wax ester molar ratio was 50.

| **FEED Composition, C₁₂ C₁₄ wax ester (WE)** | |
|---|---|
| **Compound** | **Amount, wt.%** |
| 10:10 WE | 0.1 |
| 10:10 ether | 0.0 |
| 11:12 WE | 0.0 |
| 12:12 WE | 53.3 |
| 12:12 ether | 0.0 |
| 12:14 +14:12 WE | 36.9 |
| 14:14 WE | 8.7 |
| 16:12 WE | 0.0 |
| Cis alcohol | 1.0 |

Hydrogenolysis was carried out according to the following reaction scheme:

**R-COOH + R'CH₂OH → R-CO-O-R' + 2H₂ → RCH₂OH + R'-OH**

Product samples were collected every 24 hours on stream at temperatures of 170°C, 180°C, 190°C, 200°C, and/or 220°C, respectively. These samples were analyzed by an off-line Agilent 6890 GC equipped with Quadrex Carbowax 20 M column (75 m x 0.32 mm x 0.25 µm) and flame ionization detector. C₁₂, C₁₄, C₁₆, and Cis fatty alcohols are the desired product while hydrocarbons (*e.g.*, dodecane, hexadecane) are side products. No other products were detected in any appreciable quantity.

As shown in Table 2, the exemplary catalysts 1A, 1B, and 5 showed an overall improved %conversion of wax ester at temperatures ranging from 170°C to 220°C compared to the reference copper catalyst containing silica. The exemplary copper catalysts showed an average increase in %conversion of the wax ester compared to the reference copper catalyst of approximately 14% at 170°C, 24% at 180°C, and 13% at 190°C, respectively. As shown in FIG. 1, the exemplary catalyst 5 showed an increase in %conversion of the wax ester compared to the reference copper catalyst of 33% at 170°C, 28% at 180°C, 9% at 190°C, 8% at 200°C, and 0.4% at 220°C, respectively. As shown in Table 3, exemplary catalyst 5 also showed a lower %conversion to the hydrocarbon by-product at 200°C and 220°C, respectively. Accordingly, the exemplary catalyst of the present technology exhibits a higher % conversion at lower temperatures over the comparative commercial reference catalyst.

**Table 2.**

| **Examples** | **Conversion, %** | | | | |
|---|---|---|---|---|---|
| | **170°C** | **180°C** | **190°C** | **200°C** | **220°C** |
| *Ref. | 47.9 | 54.7 | 71.3 | 82 | 90.9 |
| 1A | 61 | | 82.5 | | |
| 1B | 51.1 | 73.5 | 76.4 | | |
| 5 | 63.7 | 69.8 | 77.8 | 88.3 | 91.3 |

| | | | | | |
|---|---|---|---|---|---|
| *Reference copper and silica catalyst. | | | | | |

**Table 3.**

| **Example Catalysts** | **Hvdrocarbon Make, %** | |
|---|---|---|
| | **200°C** | **220°C** |
| *Ref. | 0.06 | 0.23 |
| 5 | 0.03 | 0.17 |

| | | |
|---|---|---|
| *Reference copper and silica catalyst. | | |

**EXAMPLE 7:** Fatty Acid Methyl Ester Hydrogenolysis Testing in a gas phase process. For gas phase methyl ester hydrogenolysis catalyst testing, 15 cc of catalyst (extrudates) was loaded into a 0.9" ID stainless steel fixed-bed down flow reactor. An equal volume of inert 28 X 48 mesh α-alumina granules was loaded with the catalyst and served as interstitial packing. The reactor was also equipped with a 3/16" thermowell that housed six thermocouples, one at the inlet and five spaced equally throughout the catalyst bed which was approximately 6" in length. The reactor and heated via furnace jacketed around the reactor tube.

Once loaded, the reactor was purged with 15 slph N₂ for approximately 1 hour to remove air and subsequently heated to 190 °C under flowing N₂. The catalyst was activated by introducing H₂ in a stepwise fashion at atmospheric pressure. Once the reduction was complete, the temperature was stabilized at 210°C, and the reactor was pressurized from atmospheric pressure to 435 psig.

After pressurization, with the temperature stable at 210°C, the hydrogen flow rate was adjusted to 800 slph. The commercial methyl feed (C₁₂-C₁₄ methyl ester) was then pumped into the reactor at a rate of 32.5 g/hr to give an overall LHSV of about 2.5 hr⁻¹. The hydrogen: methyl ester molar ratio was 250.

| **FEED Composition, C₁₂ C₁₄ methyl ester (ME)** | |
|---|---|
| **Compound** | **Amount, wt.%** |
| C₈ ME | 0.0 |
| C₁₀ ME | 0.3 |
| C₁₂ ME | 73.7 |
| C₁₄ ME | 25.3 |
| C₁₆ ME | 0.6 |
| C₁₈ (18:0) ME | 0.0 |
| C₁₈ (18:1) ME | 0.0 |
| C₁₈ (18:2) ME | 0.0 |
| C₂₄ wax ester | 0.0 |

Hydrogenolysis was carried out according to the following reaction scheme:

R-CO-O-Me + 2H₂ → RCH₂OH + MeOH

Product samples were collected every 24 hours on stream at temperatures of 210°C and 230°C, respectively. These samples were analyzed by an off-line Agilent 6890 GC equipped with Quadrex Carbowax 20 M column (75 m x 0.32 mm x 0.25 µm) and flame ionization detector. C₁₂, C₁₄, C₁₆, and C₁₈ fatty alcohols are the desired product while hydrocarbons (*e.g.*, dodecane, tetradecane and hexadecane) are side products. No other products were detected in any appreciable quantity.

As shown in Table 4, exemplary catalyst 5 showed comparative gas phase hydrogenolysis %conversion of methyl ester to the fatty alcohol product at 210°C to 230°C compared to the reference copper catalyst containing silica. Similarly, exemplary catalyst 5 showed low %conversion of the methyl ester to the hydrocarbon by-product compared to the reference copper catalyst containing silica.

| **Catalyst** | **Temp., °C** | **Conversion, %** | **Hydrocarbon concentration, %** |
|---|---|---|---|
| *Ref. | 210 | 90.9 | 0.1 |
| | 230 | 96.4 | 0.46 |
| 5 | 210 | 91.2 | 0.27 |
| | 230 | 95.5 | 0.38 |

| | | | |
|---|---|---|---|
| *Reference copper and silica catalyst. | | | |

As shown in the examples above, the catalysts of the present technology surprisingly show improved liquid phase hydrogenolysis %conversion of wax ester feeds to the fatty alcohol products and lower conversion to the hydrocarbon by-product over commercial copper catalysts containing silica. In addition, the catalysts of the present technology surprisingly show comparative gas phase hydrogenolysis %conversion of methyl ester feeds to the fatty alcohol product and low conversion to the hydrocarbon by-product comparable with commercial copper catalysts containing silica.

While certain embodiments have been illustrated and described, it should be understood that changes and modifications may be made therein in accordance with ordinary skill in the art without departing from the technology in its broader aspects as defined in the following claims.

## Claims

1. A hydrogenolysis catalyst comprising:
a catalytic component comprising copper oxide, manganese oxide, and aluminum oxide; and
a binder comprising a zirconium component,
wherein:
the catalyst comprises copper oxide in an amount from 45 wt% to 55 wt%,
the catalyst comprises less than 1.5 wt.% of silicon or an oxide thereof, based on the total weight of the hydrogenolysis catalyst, and
wherein the zirconium component is present in an amount from 4 wt% to 15 wt% by weight of the hydrogenolysis catalyst.

2. The hydrogenolysis catalyst of claims 1, wherein the manganese oxide is present in an amount from 5 wt% to 12 wt% by weight of the hydrogenolysis catalyst.

3. The hydrogenolysis catalyst of any one of claims 1 or 2, wherein the aluminum oxide is present in an amount from 15 wt% to 40 wt% by weight of the hydrogenolysis catalyst.

4. The hydrogenolysis catalyst of any one of claims 1-3, wherein zirconium component is zirconium oxide.

5. The hydrogenolysis catalyst of claims 1, wherein the zirconium component is present in an amount from about 5 wt% to about 12 wt% by weight of the hydrogenolysis catalyst.

6. The hydrogenolysis catalyst of any one of claims 1-5, wherein the binder further comprises alumina.

7. The hydrogenolysis catalyst of any one of claims 1-6, wherein the hydrogenolysis catalyst further comprises an alkali metal component.

8. The hydrogenolysis catalyst of any one of claims 1-7, wherein the hydrogenolysis catalyst is in an unreduced form and exhibits an X-ray powder diffraction profile with 2θ peaks at 31.1°, 36.0°, 36.8°, 38.8°, 44.6°, 48.8°, 61.7°, 64.9°, 68.1° is in an unreduced form and exhibits an X-ray powder diffraction profile with 2θ peaks.

9. The hydrogenolysis catalyst of any one of claims 1-8wherein the hydrogenolysis catalyst is a calcined and extruded hydrogenolysis catalyst.

10. The hydrogenolysis catalyst of any one of claims 1-9, wherein the hydrogenolysis catalyst exhibits a pore volume of greater than 0.25 cm³/g, a packed bulk density of 0.8 g/cm³ to 1.5 g/cm³, a Brunauer-Emmett-Teller ("BET") surface area from 15 m²/g to 70 m²/g

11. A method of preparing a calcined hydrogenolysis catalyst according to any of the claims 1 to 10, the method comprising:
mixing a catalytic component with a binder system and water to obtain a material mixture;
forming the material mixture to obtain a formed material mixture;
calcining the formed material mixture at a temperature, and for a time, sufficient to cure form the calcined hydrogenolysis catalyst;
wherein:
the catalytic component comprises copper oxide, manganese oxide, and aluminum oxide;
the binder system comprises a zirconium component;
the calcined hydrogenolysis catalyst comprises copper oxide in an amount from 45 wt% to 55 wt%;
the calcined hydrogenolysis catalyst comprises less than 1.5 wt.% of silicon or an oxide thereof, based on the total weight of the hydrogenolysis catalyst, and
wherein the zirconium component is present in an amount from 4 wt% to 15 wt% by weight of the hydrogenolysis catalyst.

12. The method of claim 11, wherein the catalytic component comprises pre-calcined powder comprising copper oxide, manganese oxide, and aluminum oxide.

13. The method of claim 12, wherein the pre-calcined powder was calcined at a temperature from 400°C to 800°C.

14. A method of conducting hydrogenolysis of a fatty acid ester, the method comprising contacting the fatty acid ester with a hydrogenolysis catalyst according to any of the claims 1-10.

15. The method of claim 14, wherein the fatty acid ester is represented by Formula I:
R¹-CO-OR² (I),
wherein
R¹ and R² are each independently a branched or unbranched C₁ to C₂₄ alkyl or a branched or unbranched C₂ to C₂₄ alkenyl.

## Patentansprüche

1. Hydrogenolysekatalysator, umfassend:
eine katalytische Komponente, umfassend Kupferoxid, Manganoxid und Aluminiumoxid; und
ein Bindemittel, umfassend eine Zirkoniumkomponente,
wobei:
der Katalysator Kupferoxid in einer Menge von 45 Gew.-% bis 55 Gew.-% umfasst,
der Katalysator weniger als 1,5 Gew.-% Silizium oder ein Oxid davon umfasst, basierend auf dem Gesamtgewicht des Hydrogenolysekatalysators, und
wobei die Zirkoniumkomponente in einer Menge von 4 Gew.-% bis 15 Gew.-% des Hydrogenolysekatalysators vorhanden ist.

2. Hydrogenolysekatalysator nach Anspruch 1, wobei das Manganoxid in einer Menge von 5 Gew.-% bis 12 Gew.-%, bezogen auf das Gewicht des Hydrogenolysekatalysators, vorhanden ist.

3. Hydrogenolysekatalysator nach einem der Ansprüche 1 oder 2, wobei das Aluminiumoxid in einer Menge von 15 Gew.-% bis 40 Gew.-% des Gewichts des Hydrogenolysekatalysators vorhanden ist.

4. Hydrogenolysekatalysator nach einem der Ansprüche 1 bis 3, wobei die Zirkoniumkomponente Zirkoniumoxid ist.

5. Hydrogenolysekatalysator nach Anspruch 1, wobei die Zirkoniumkomponente in einer Menge von etwa 5 Gew.-% bis etwa 12 Gew.-% des Gewichts des Hydrogenolysekatalysators vorhanden ist.

6. Hydrogenolysekatalysator nach einem der Ansprüche 1 bis 5, wobei das Bindemittel ferner Tonerde umfasst.

7. Hydrogenolysekatalysator nach einem der Ansprüche 1 bis 6, wobei der Hydrogenolysekatalysator ferner eine Alkalimetallkomponente umfasst.

8. Hydrogenolysekatalysator nach einem der Ansprüche 1 bis 7, wobei der Hydrogenolysekatalysator in einer nicht reduzierten Form vorliegt und ein Röntgenpulverbeugungsprofil mit 2θ Spitzen bei 31,1°, 36,0°, 36,8°, 38,8°, 44,6°, 48,8°, 61,7°, 64,9°, 68,1° in einer nicht reduzierten Form vorliegt und ein Pulverröntgenbeugungsprofil mit 2θ Spitzen aufweist.

9. Hydrogenolysekatalysator nach einem der Ansprüche 1 bis 8, wobei der Hydrogenolysekatalysator ein kalzinierter und extrudierter Hydrogenolysekatalysator ist.

10. Hydrogenolysekatalysator nach einem der Ansprüche 1 bis 9, wobei der Hydrogenolysekatalysator ein Porenvolumen von mehr als 0,25 cm³/g, eine Schüttdichte von 0,8 g/cm³ bis 1,5 g/cm³, einen Oberflächenbereich nach Brunauer-Emmett-Teller ("BET") von 15 m²/g bis 70 m²/g aufweist.

11. Verfahren zum Zubereiten eines kalzinierten Hydrogenolysekatalysators nach einem der Ansprüche 1 bis 10, das Verfahren umfassend:
Mischen einer katalytischen Komponente mit einem Bindemittelsystem und Wasser, um eine Materialmischung zu erhalten;
Formen der Materialmischung, um eine geformte Materialmischung zu erhalten;
Kalzinieren der gebildeten Materialmischung bei einer Temperatur und für eine Zeit, die ausreichen, um den kalzinierten Hydrogenolysekatalysator zu härten;
wobei:
die katalytische Komponente Kupferoxid, Manganoxid und Aluminiumoxid umfasst;
das Bindemittelsystem eine Zirkoniumkomponente umfasst;
der kalzinierte Hydrogenolysekatalysator Kupferoxid in einer Menge von 45 Gew.-% bis 55 Gew.-% umfasst;
der kalzinierte Hydrogenolysekatalysator weniger als 1,5 Gew.-% Silizium oder ein Oxid davon umfasst, basierend auf dem Gesamtgewicht des Hydrogenolysekatalysators, und
wobei die Zirkoniumkomponente in einer Menge von 4 Gew.-% bis 15 Gew.-% des Hydrogenolysekatalysators vorhanden ist.

12. Verfahren nach Anspruch 11, wobei die katalytische Komponente ein vorkalziniertes Pulver umfasst, das Kupferoxid, Manganoxid und Aluminiumoxid umfasst.

13. Verfahren nach Anspruch 12, wobei das vorkalzinierte Pulver bei einer Temperatur von 400 °C bis 800 °C kalziniert wurde.

14. Verfahren zum Durchführen der Hydrogenolyse eines Fettsäureesters, wobei das Verfahren das Inkontaktbringen des Fettsäureesters mit einem Hydrogenolysekatalysator nach einem der Ansprüche 1 bis 10 umfasst.

15. Verfahren nach Anspruch 14, wobei der Fettsäureester durch die Formel I dargestellt wird:
R¹CO-OR² (I),
wobei
R¹ und R² jeweils unabhängig ein verzweigtes oder unverzweigtes C₁- bis C₂₄-Alkyl oder ein verzweigtes oder unverzweigtes C₂- bis C₂₄-Alkenyl sind.

## Revendications

1. Catalyseur d'hydrogénolyse comprenant :
un composant catalytique comprenant de l'oxyde de cuivre, de l'oxyde de manganèse, et de l'oxyde d'aluminium ; et
un liant comprenant un composant de zirconium,
dans lequel :
le catalyseur comprend de l'oxyde de cuivre en une quantité allant de 45 % en poids à 55 % en poids,
le catalyseur comprend moins de 1,5 % en poids de silicium ou d'un oxyde de celui-ci, sur la base du poids total du catalyseur d'hydrogénolyse, et
dans lequel le composant de zirconium est présent en une quantité allant de 4 % en poids à 15 % en poids, en poids du catalyseur d'hydrogénolyse.

2. Catalyseur d'hydrogénolyse selon la revendication 1, dans lequel l'oxyde de manganèse est présent en une quantité allant de 5 % en poids à 12 % en poids, en poids du catalyseur d'hydrogénolyse.

3. Catalyseur d'hydrogénolyse selon l'une quelconque des revendications 1 ou 2, dans lequel l'oxyde d'aluminium est présent en une quantité allant de 15 % en poids à 40 % en poids, en poids du catalyseur d'hydrogénolyse.

4. Catalyseur d'hydrogénolyse selon l'une quelconque des revendications 1 à 3, dans lequel le composant de zirconium est l'oxyde de zirconium.

5. Catalyseur d'hydrogénolyse selon la revendication 1, dans lequel le composant de zirconium est présent en une quantité allant d'environ 5 % en poids à environ 12 % en poids, en poids du catalyseur d'hydrogénolyse.

6. Catalyseur d'hydrogénolyse selon l'une quelconque des revendications 1 à 5, dans lequel le liant comprend en outre de l'alumine.

7. Catalyseur d'hydrogénolyse selon l'une quelconque des revendications 1 à 6, dans lequel le catalyseur d'hydrogénolyse comprend en outre un composant de métal alcalin.

8. Catalyseur d'hydrogénolyse selon l'une quelconque des revendications 1 à 7, dans lequel le catalyseur d'hydrogénolyse est sous forme non réduite et présente un profil de diffraction des rayons X sur poudre avec des pics 2θ à 31,1°, 36,0°, 36,8°, 38,8°, 44,6°, 48,8°, 61,7°, 64,9°, 68,1° est sous forme non réduite et présente un profil de diffraction des rayons X sur poudre avec des pics 2θ.

9. Catalyseur d'hydrogénolyse selon l'une quelconque des revendications 1 à 8, dans lequel le catalyseur d'hydrogénolyse est un catalyseur d'hydrogénolyse calciné et extrudé.

10. Catalyseur d'hydrogénolyse selon l'une quelconque des revendications 1 à 9, dans lequel le catalyseur d'hydrogénolyse présente un volume de pores supérieur à 0,25 cm³/g, une masse volumique apparente tassée de 0,8 g/cm³ à 1,5 g/cm³, une surface Brunauer-Emmett-Teller (« BET ») allant de 15 m²/g à 70 m²/g

11. Procédé de préparation d'un catalyseur d'hydrogénolyse calciné selon l'une quelconque des revendications 1 à 10, le procédé comprenant :
le mélange d'un composant catalytique avec un système de liant et de l'eau pour obtenir un mélange de matériaux ;
la mise en forme du mélange de matériaux pour obtenir un mélange de matériaux mis en forme ;
la calcination du mélange de matériaux mis en forme à une température et pendant une durée suffisantes pour mettre en forme par durcissement le catalyseur d'hydrogénolyse calciné ;
dans lequel :
le composant catalytique comprend de l'oxyde de cuivre, de l'oxyde de manganèse, et de l'oxyde d'aluminium ;
le système de liant comprend un composant de zirconium ;
le catalyseur d'hydrogénolyse calciné comprend de l'oxyde de cuivre en une quantité allant de 45 % en poids à 55 % en poids ;
le catalyseur d'hydrogénolyse calciné comprend moins de 1,5 % en poids de silicium ou d'un oxyde de celui-ci, sur la base du poids total du catalyseur d'hydrogénolyse, et
dans lequel le composant de zirconium est présent en une quantité allant de 4 % en poids à 15 % en poids, en poids du catalyseur d'hydrogénolyse.

12. Procédé selon la revendication 11, dans lequel le composant catalytique comprend une poudre précalcinée comprenant de l'oxyde de cuivre, de l'oxyde de manganèse, et de l'oxyde d'aluminium.

13. Procédé selon la revendication 12, dans lequel la poudre précalcinée a été calcinée à une température allant de 400 °C à 800 °C.

14. Procédé de conduite d'hydrogénolyse d'un ester d'acide gras, le procédé comprenant la mise en contact de l'ester d'acide gras avec un catalyseur d'hydrogénolyse selon l'une quelconque des revendications 1 à 10.

15. Procédé selon la revendication 14, dans lequel l'ester d'acide gras est représenté par la Formule I :
R¹-CO-OR² (I),
dans laquelle
R¹ et R² sont chacun indépendamment un alkyle en C₁ à C₂₄ ramifié ou non ramifié ou un alcényle en C₂ à C₂₄ ramifié ou non ramifié.
